# EUROPEAN PATENT APPLICATION

(11) **EP 0 553 428 A2**
(43) Date of publication of application: **04.08.1993**
(21) Application number: 92120158.8
(22) Date of filing: 11.12.1992
(51) Int. Cl.: C12N 15/12, A01K 67/027, C12N 15/85, A61K 37/02, C12Q 1/02, C12Q 1/68

(54) **DNA 5' to an untranslated region upstream of a gene encoding a growth hormone receptor. Use as a sex promoter**

(30) Priority: 31.12.1991 US 816287; 10.09.1992 US 945290
(71) Applicant: AMERICAN CYANAMID COMPANY, Wayne, NJ 07470-8426 (US)
(72) Inventor: Baumbach, William Robert, Hopewell, New Jersey (US); Logan, John Steele, Robbinsville, New Jersey (US)
(74) Representative: Wächtershäuser, Günter, Prof. Dr.

(57) **Abstract**

The present invention relates to an isolated nucleic acid sequence which, as a promoter, is regulated in a developmental- and sex-specific manner. The sequence is useful in DNA constructs, in combination with a gene of interest, to achieve developmental and sex-specific expression of the gene in transgenic animals.

## Description

### FIELD OF THE INVENTION

The present invention provides novel isolated DNA sequences. More specifically, the invention provides isolated regulatory DNA sequences which are capable of controlling gene expression in a sex- and developmental-specific manner.

### BACKGROUND OF THE INVENTION

All genes must be under strict control as to their use as templates for transcription of RNA. This control is the primary regulatory point which determines the eventual amount of final product produced by any cell, whether that product is protein or RNA itself. Other levels of control occur at subsequent nuclear steps in RNA processing such as splicing, degradation, or transport. Cytoplasmic events like translation of RNA into protein and the post-translational processing of proteins offer additional control. Transcriptional control is thought to be determined by a variety of polypeptide factors which specifically interact with sequence motifs of the DNA surrounding (or contained within) any particular gene. For the most part, sequences that effect transcription are found 5' of the structural gene (although such sequences can be found 3' of the gene or within the gene itself). Thus, each particular species of mRNA will have a unique environment 5' of the start site for transcription, consisting of a specific DNA sequence. The interaction of those sequences with transcription factors and RNA polymerase II determines for the most part how any species of mRNA is transcribed in a particular tissue, sex, developmental or temporal period of the animal, and at what levels it is produced.

The rat growth hormone receptor (GHR) is translated from at least two different mRNA species, termed Rat 1 and Rat 2. The major difference between these mRNAs is found in the 5' untranslated regions which are alternatively spliced to Exon 2 of the GHR (where the translation initiation codon is found). Thus there are at least two alternative first exons for the GHR mRNA, and the genomic DNA sequences 5' of the initiation site for transcription of these messages are likely to contain sequences responsible for controlling this transcription.

Transgenic animals contain exogenous pieces of DNA integrated into their genomic structure. This exogenous DNA consists of the sequence of a particular gene(s) of interest, either with or without introns, along with surrounding sequences. The expression of this DNA is dependent largely upon sequences found 5' of the gene of interest. Since the integration of a transgene into the genome is generally random, this 5' region must be included as an integral part of the cloned DNA which is introduced into a transgenic animal. Thus, each potential promoter sequence (5' region) may have particular uses for directing the expression of transgenes.

Each transgenic animal has preferred expression patterns for its particular transgene, and it is seldom desirable that the transgene be consistently expressed at all times during the animal's life. For instance, growth hormone should be overexpressed for maximum effect in livestock over a fixed period of time, such as the six-week finishing period in swine prior to slaughter. Expression of the exogenous GH gene (transgene) at other times can lead to a variety of unwanted side effects such as weight loss, reproductive deficiencies or chronic physical symptoms like arthritis. Thus, adequate controlling elements are essential for improved performance due to transgene expression in transgenic livestock. The present invention now provides a means by which the expression of a given gene of interest can be controlled in a developmental and/or sex-specific manner.

### SUMMARY OF THE INVENTION

The present invention relates to an isolated nucleic acid sequence comprising a region 5' to an untranslated region upstream of a gene encoding a growth hormone receptor. In one specific embodiment exemplified in the present application by the rat sequence, the "untranslated region" refers to the untranslated Exon 1 and Intron 1 of the growth hormone receptor gene. In vivo, the sequence is characterized by delayed expression (until adolescence) of the gene under its control; and significant expression only in females and castrated males. The nucleic acid sequence is particularly useful as a controlling element in a DNA construct, operably linked to a gene of interest to be expressed in a transgenic animal. Such constructs can be incorporated into the germ line of an animal but will not be expressed in early development. The delay in expression of a transgene can be useful in animals being used as screening tools, since it will permit the use of toxic or detrimental gene products, such as disease model genes, because they will not be expressed early in development when they may be lethal. Further, the transgenic germ line will remain intact because normal males will not express a toxic or detrimental phenotype, thereby preserving their reproductive fitness. Also, the phenotype can be induced in males by castration. An introduced phenotype advantageous to animal growth efficiency in transgenic livestock will thus be expressed not only in females, but also in castrated males, which is to the benefit of livestock producers.

It is also determined that the activity of the promoter is related to the presence of estrogen. In the presence of estrogen, the promoter directs expression of the gene under its control. Therefore, exogenous administration of estrogen to a transgenic animal carrying the gene of interest can induce production of the gene product. Moreover, as the regulatory element that appears to control expression of the growth hormone receptor in vivo, a similar method can also be used to control the expression of the native growth hormone receptor in animals; this can be particularly useful in birds, particularly in chickens, in which the receptor is not expressed until late in development, i.e., at about the time at which the birds are usually killed; and therefore, exogenous administration of growth hormone has no effect in these birds. Administration of estrogen prior to or simultaneously with administration of growth hormone can be used to enhance growth in chickens.

### BRIEF DESCRIPTION OF THE FIGURES

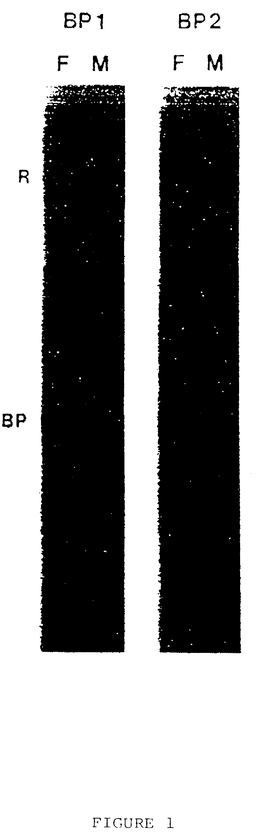

**Figure 1**. Northern analysis of RNA from male and female rat liver. Probes are 5'BP1 and 5'BP2. Each lane contains 3 ug of poly(a) RNA from adult (8 week old) rat liver, male (M) or female (F). Positions of GH receptor (R) and GH binding protein (BP) RNA are indicated.

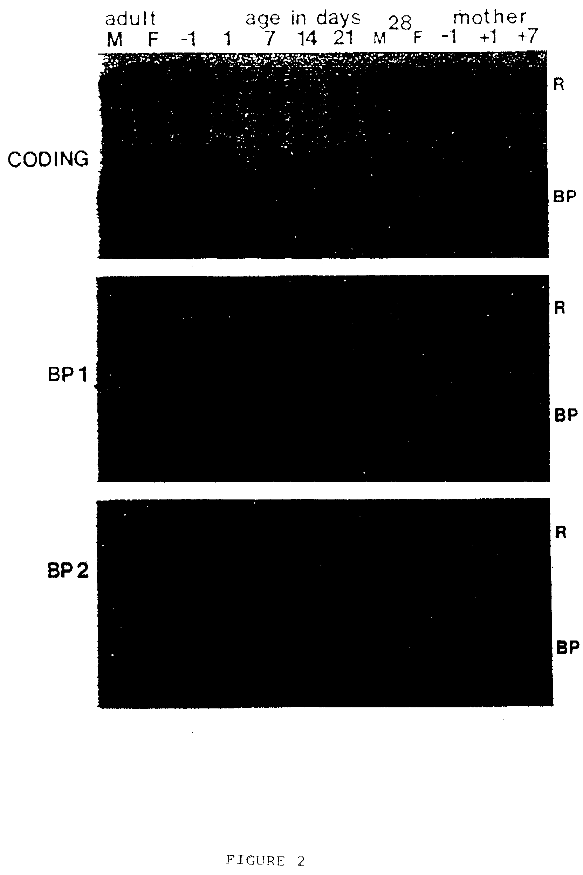

**Figure 2**. Northern analysis of RNA from rat liver at various stages of development. Probes are rat 1-20 (coding region), 5'BP1 and 5'BP2. Each lane contains 3 ug of poly(A) RNA from adult (8 week old) male (M) and female (F); pooled male and female fetal (-1), newborn (1), one week (7), two week (14), three week (21) liver; 4 week male (M) and female (F) liver; and pregnant female (9 week old) one day before (-1), 1 day after (+1), and one week after (+7) birth.

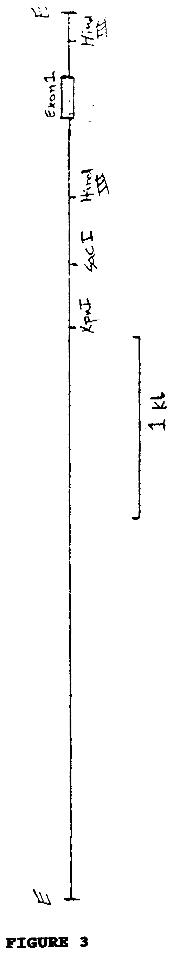

**Figure 3**. Restriction map of a 4.9 Kb fragment of pRAT1-E, a subclone of the rat genomic clone called λ Rat 1. The 5' to 3' orientation of this map reflects the correct transcriptional orientation of Exon 1 of RatBP1. The region representing Exon 1 of RatBP1 is boxed.

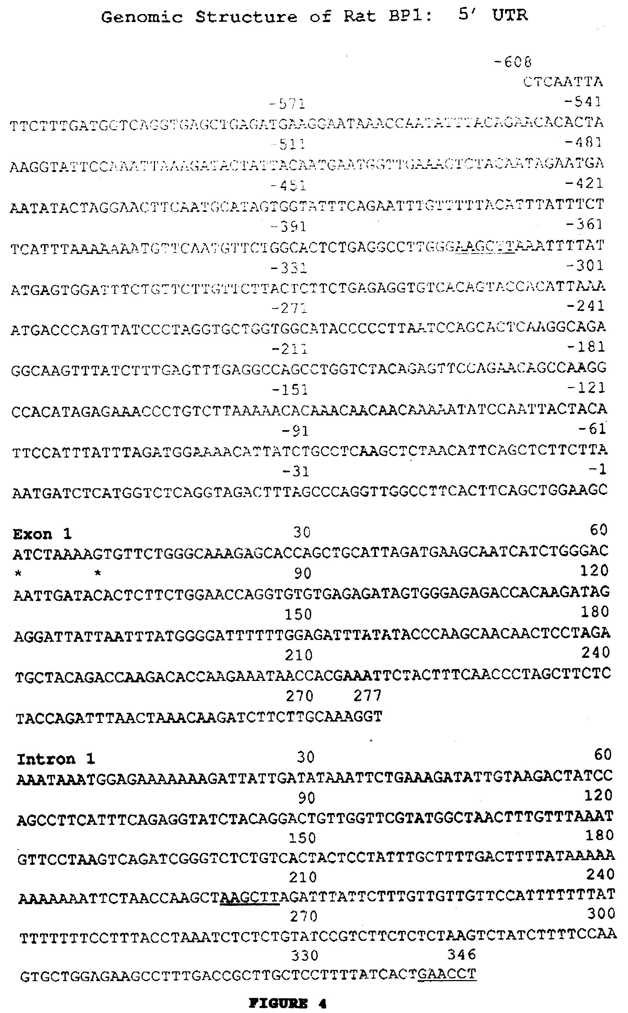

**Figure 4**. Sequence analysis of a portion of pRat1-E, which contains the first exon of the rat growth hormone binding protein cDNA RatBP1. The region of exon 1 is shown, with stars below the putative transcriptional start sites based on primer extension analysis. Also shown are the first 446 nucleotides on Intron 1 and the 608 nucleotides upstream of the first putative transcriptional start site. The HindIII and EcoRI sites seen in Figure 3 are underlined.

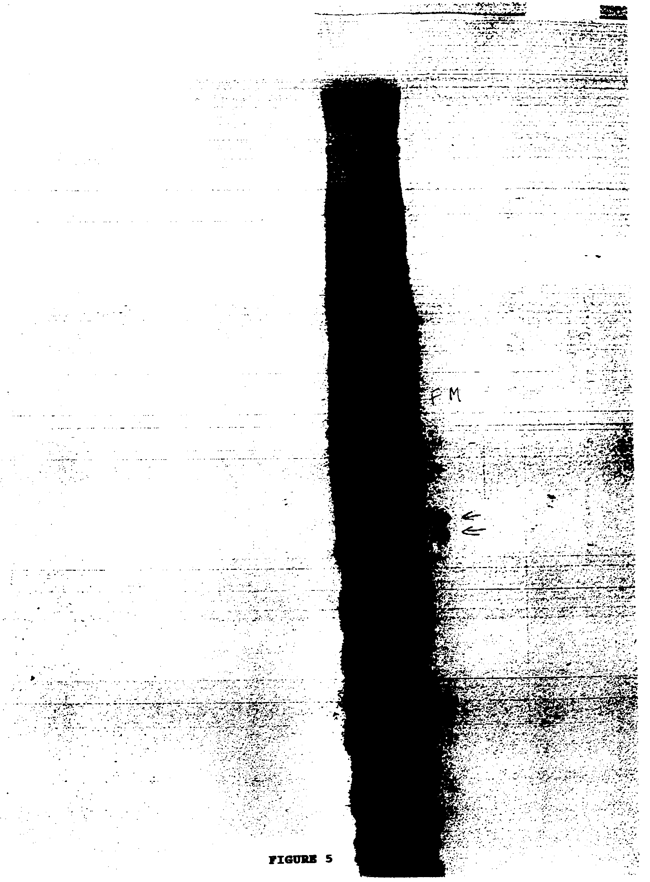

**Figure 5**. Primer extension analysis of RatBP1. Reverse transcription of female rat liver mRNA is primed with the oligonucleotide BP-1B (-101 to -130). DNA sequence reactions of pRAT1-HH (-375 to +201)are performed using the same primer, and transcriptional start sites are determined by comparing the two on a 6% polyacrylamide-urea gel. Arrows highlight the transcriptional start sites.

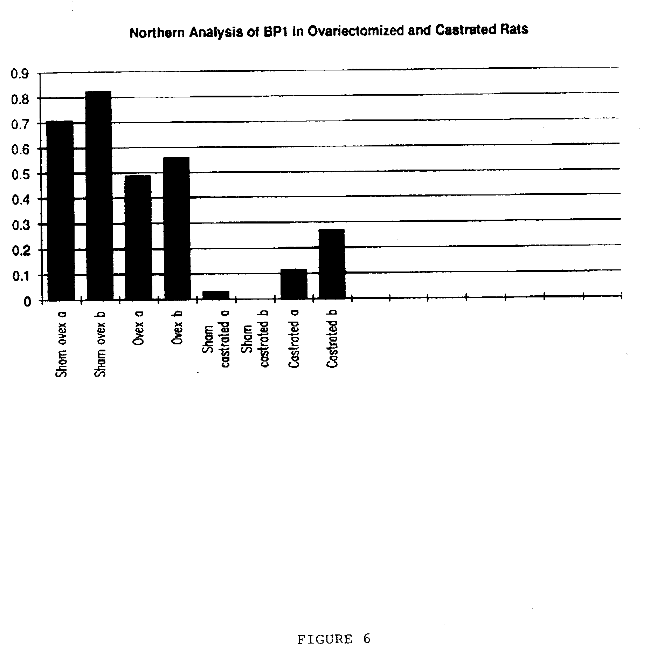

**Figure 6**. Northern Analysis of GHBP-BP1 RNA in rat liver. 3 µg of poly(A) RNA is run per lane. Rats are ovariectomized (ovex) or castrated. mRNA from the livers of these animals is compared with mRNA from sham-operated animals. Units are arbitrary signal units that have been normalized to constant levels of GAPDH mRNA.

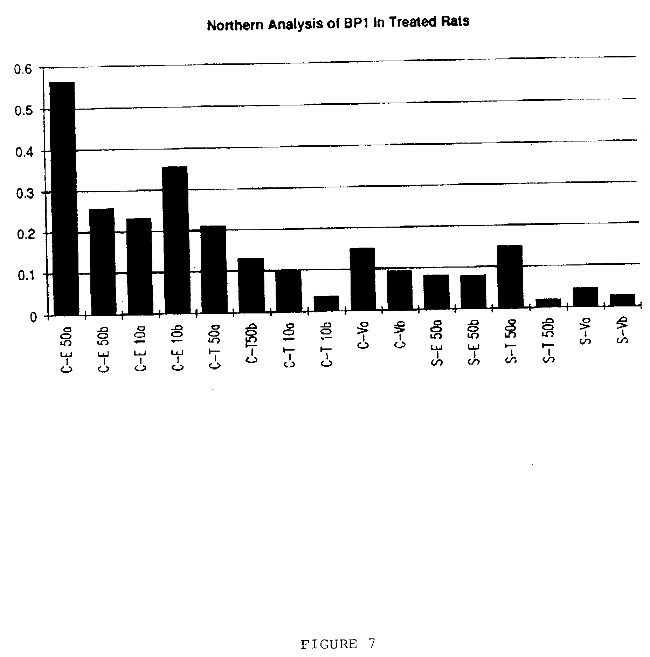

**Figure 7**. Northern analysis of GHBP-BP1 RNA in rat liver. 3 µg of poly(A) RNA is run per lane. Castrated rats and sham-castrated rats are operated upon at 6 weeks of age and treated with estrogen (50 µg/day or 10 µg/day), testosterone (50 µg/day or 10 µg/day) or vehicle for 3 days. Rats are sacrificed on day 3. Units are arbitrary signal units that have been normalized to constant GAPDH RNA levels. c, castrated; s, sham; E, estrogen, T, testosterone; V, vehicle.

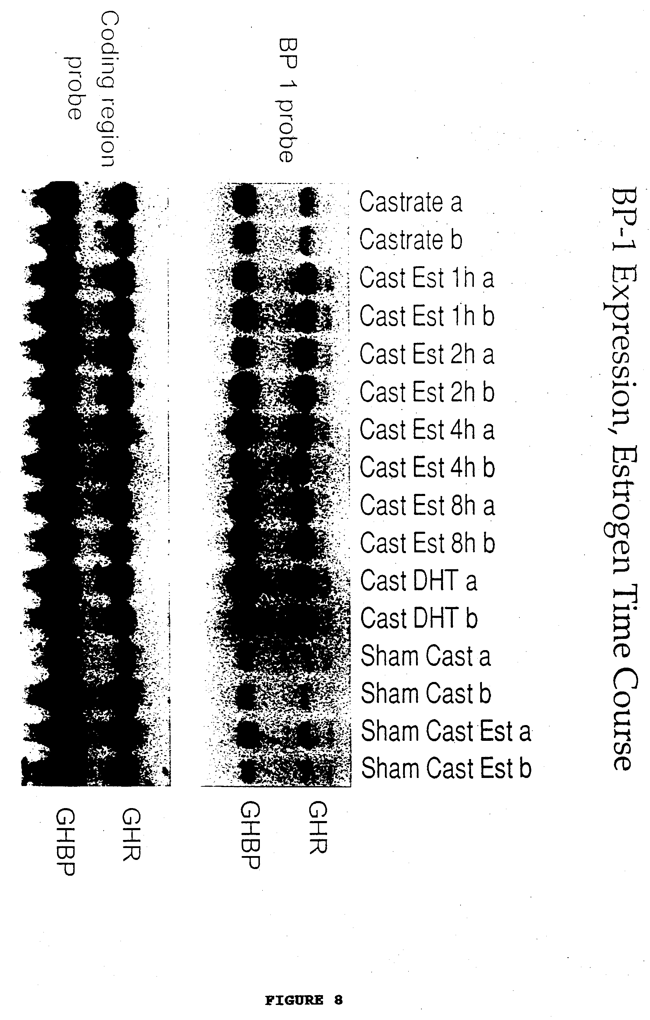

**Figure 8**. Northern analysis of castrated rat liver, estrogen treatments. Four µg poly(A) RNA from rat livers are fractionated on a 1% formaldehyde-agarose gel. Probes are indicated at left; GHR and GHBP bands are indicated at right; est, estrogen, a and b, individual animals within each group.

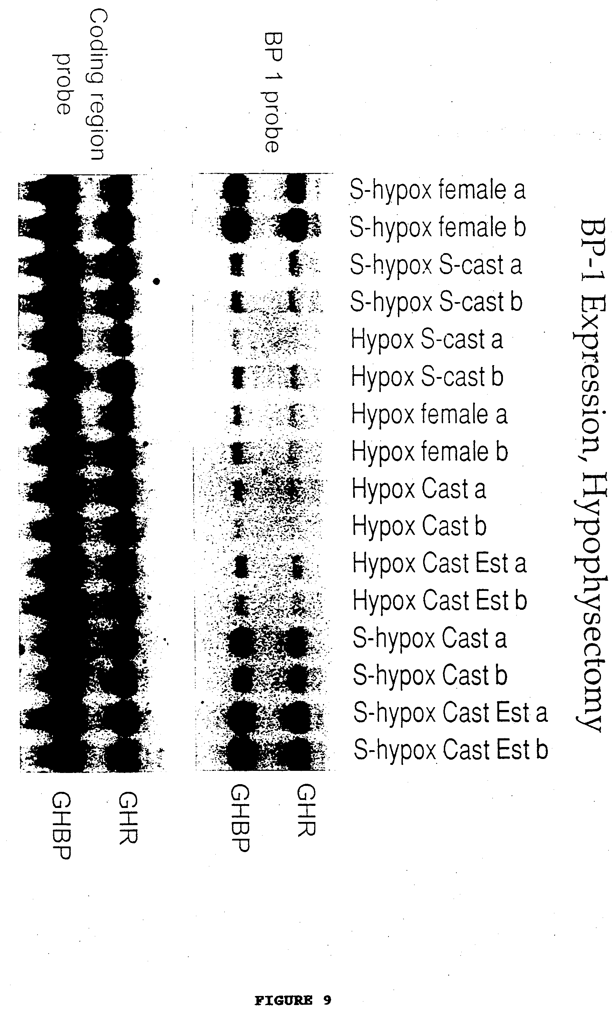

**Figure 9**. Northern analysis of hypox rat liver. Gel and probes are as in Figure 8. S-hypox, sham hypophysectomy; s-cast, sham castration; a and b, individual animals within each group.

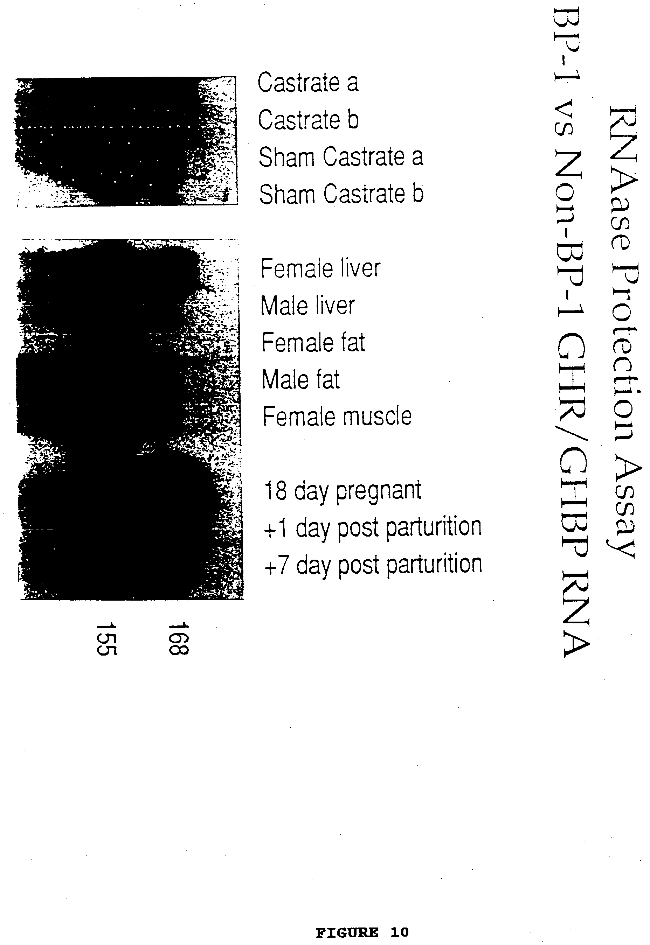

**Figure 10**. RNAase protection assay, RNA from rat tissues. Protected probe is run on a 6% denaturing polyacrylamide-urea gel. Size of bands shown at right: 168, BP-1 RNA, 155, all other GHR/GHBP RNA.

### DETAILED DESCRIPTION OF THE INVENTION

The rat growth hormone is translated from at least two different RNA species (hereafter referred to as Rat 1 and Rat 2). These two species differ primarily in the 5' untranslated region. The 5, untranslated region in vivo is alternatively spliced to exon 2 of the growth hormone receptor. Since the genomic DNA sequences 5' of the transcription initiation site (located in Exon 2) is considered likely to contain the sequences controlling transcription, investigation of the nature of the differences in these two 5' regions is initiated. Two cDNA clones, referred to as pRat1 and pRat6, of the rat growth hormone binding protein (which is a product of alternative splicing of the growth hormone receptor gene), are used to prepare DNA probes based on the respective 5' untranslated regions of each clone. The different 5' untranslated regions are referred to as BP1 and BP2, respectively, and their respective probes are referred to as 5'BP1 and 5'BP2. These two probes are individually hybridized to mRNA derived from rat liver. The 5'BP1 probe hybridizes with two species of mRNA from adult female rat liver, but does not hybridize with a corresponding species in male adult rat liver; however, 5'BP2 hybridizes with the same two species in both male and female livers. This suggests that specific sequences control the transcription of a class of mRNA derived from 5'BP1 in a sex-specific manner. As these control sequences do not appear to affect transcription of mRNA hybridizing to 5'BP2, it can be deduced that the control sequences are cis-acting and are found 5' to the genomic sequence containing the 5' untranslated region found in pRat1.

The 5'BP1 and 5'BP2 clones are again used as probes, with a third probe, Rat1-20, which is derived from the coding region shared by the rat growth hormone receptor and binding protein, in Northern analysis of polyA(+) RNA preparations from rat livers. The mRNA levels indicated by hybridization of Rat1-20 probe represent the total amount of receptor and binding protein mRNA, including mRNA containing various 5' or 3' untranslated regions while levels of mRNA encoding receptor and binding protein that hybridize to either 5'BP1 or 5'BP2 probes each represent a subset of that total mRNA. As shown in Figure 2, as liver development proceeds from day 1 through day 28, total levels of receptor and binding protein gradually increase, with a similar pattern occurring with both 5'BP2 and Rat1-20 probes. In contrast, the expression seen with the 5'BP1 probe is much less through day 28, but in adult female rats, the level is very high. This indicates that the expression of the 5'BP is more strongly developmentally regulated that the total developmental regulation of receptor and binding protein message. It is first detected at about four weeks of age in both males and females, and in females rapidly reaches the high levels seen in the adult; on the other hand, the low level seen in four-week old males does not appreciably increase with age. Levels of BP1 hybridizing message is uniformly high in pregnant females.

To isolate the DNA containing the control region, genomic DNA from rat liver is used to create a phage library. One clone hybridizes to the 5'BP1 probe and is analyzed further. A 5-kilobase EcoRI fragment is subcloned, and called pRat1E. This fragment contains the 5' untranslated region found in pRat1. A restriction map of fragment pRat1E is shown in Figure 3, with the sequenced region shown in Figure 4 highlighted. Inspection of the sequence reveals the following: all of the BP1 5' untranslated region is present (called Exon 1), the point at which an RNA splicing joins this region to the rest of the receptor binding protein sequence is deduced by a loss of homology to BP1, and signals the beginning of Intron 1.

The start site of transcription for Exon 1 is determined by primer extension analysis (Maniatis et al., 1989). Reverse transcription of female and male rat liver mRNA is primed by an oligonucleotide called BP1B (-101 to -130). This primer hybridizes to the RNA sequence underlined in Figure 4, and is extended by reverse transcription to the 3' end of the mRNA, indicating the transcriptional start site of these messages. Figure 5 shows two bands using female liver mRNA and nothing using male liver mRNA. In Figure 4, stars underneath the sequence denote the bases determined to be transcriptional start sites. This location is thus shown in Figure 4 as the beginning of Exon 1.

The isolated DNA useful as a regulated promoter may comprise all or a portion of the region 5' to the untranslated region upstream of the growth hormone receptor. The isolated DNA sequence may also be extended to encompass all or a portion of the 5, untranslated region as well. With specific reference to the exemplified rat DNA, as shown in Figures 3 and 4, the novel DNA isolate comprises that portion of the 4.9 Kb fragment upstream of Exon 1. In an alternate embodiment, the size of the isolate may be expanded to encompass all or a portion of Exon 1, and further may encompass all or a portion of Intron 1. It will also be recognized that smaller portions of the 4.9 Kb fragment can be identified by routine, sequential deletions of nucleotides from either end of the fragment to yield more compact isolates which retain the biological characteristics, specifically with regard to the capacity for developmental regulation, exhibited by the 4.9 Kb fragment.

It will also be recognized by those skilled in the art that the invention is not limited by the sequence defined by the rat isolate or portions thereof. The invention also encompasses corresponding isolated nucleic acid sequences from other animals as well, i.e. all or a portion of those sequences 5' to the growth hormone receptor gene in other animals, such as cattle, pig, sheep, goats, chickens, ducks, turkeys, quail and geese. These can be identified by using pRat1-E or the promoter, as depicted in Figure 4, either with or without the Exon 1 sequence, as a probe in routine hybridization reactions with nucleic acid derived from the species of interest.

The isolated DNA sequences are particularly useful as control elements in DNA constructs for use in creating transgenic animals. In brief, the novel sequence is linked to a gene of interest, for which developmentally regulated expression is desirable. For example, overexpression of growth hormone in early development can have a detrimental effect on transgenic livestock, in terms of weight loss and other abnormalities. Linking the gene encoding growth hormone to the novel promoter sequence provides a DNA construct that can be used in creating a transgenic animal in which growth hormone production can be developmentally controlled. In females, expression will begin in adolescence, and in males, expression can be induced at an appropriate time by castration. This control of expression is useful in controlling growth in any animal, but is particularly useful in growth enhancement of livestock, e.g. cattle, sheep, pigs, goats, chickens, ducks, geese, turkeys, and the like. Although a growth hormone construct represents a particularly useful embodiment, such constructs can also be used with other genes, such as oncogenes, which can be used in the study of cancer development.

Methods for creating transgenic animals have been described in U.S. Patent No. 4,736,866, the contents of which are incorporated herein by reference. Briefly, for example, in mammals, the construct is injected into the male pronucleus of a fertilized one-cell egg, prior to its fusion with the female pronucleus. The injected eggs are then transferred to pseudo-pregnant females (Wagner, PNAS USA 78:5016, 1984) and allowed to develop to term.

Methods for creating transgenic birds are also available in the art. For example, sperm-mediated transfer can be used to insert the sequence into an egg nucleus. Alternately, a viral vector, such as a retrovirus, can also be used to achieve host cell transformation. Such methods are well known in the art, e.g., Bandyopadhiyay and Temen. Mol. Cell. Biol. 4:743-748, and 749-754, 1984.

The novel DNA sequence is also useful in the creation of screening systems to identify other compounds which may be used to modulate the activity of the promoter. For example, test microorganisms such as bacteria or yeast are constructed by linking the novel promoter to a reporter gene , i.e., a gene which is capable of producing a detectable signal upon expression, e.g. β-galactosidase. Samples to be tested are contacted with the screening microorganism; the observation of a detectable signal indicates the presence of a compound that induces the promoter.

The present invention also provides a means for modulating expression of the native growth hormone receptor in vivo. It has not previously been observed that the control region for at least one species of growth hormone receptor mRNA is hormonally regulated. The data provided herein show that receptor expression is induced in the presence of estrogen. Thus, expression of the native receptor can be manipulated by administration of estrogen or estrogen-like compounds.

As the data in Example 6 further indicate, the effect of estrogen may be indirect, i.e., mediated through other hormones: hypophysectomized rats, even when treated with estrogen, fail to show induction of BP-1. These results suggest that the observed effect of estrogens may be due to a more direct action of an anterior pituitary hormone, such as ACTH, LH, FSH or TSH, or even GH, on the promoter. Preliminary data (not shown) suggest at least some involvement of GH. If GH were to be involved, this would confirm and further explain the observations of Talamantes et al. (in Mena et al., eds., Frontiers and Perspective of Prolactin Secretin: A Multidisciplinary Approach. Academic Press, NY, pp. 31-44, 1984) and Cramer et al. (Endocrinology, 131:876-882, 1992) relating to serum GH levels and GH receptor expression during pregnancy. Additional data, shown in Example 7, favor a connection with reproduction-related pituitary hormones such as FSH and LH: these results not only confirm the preferential expression of BP-1 in female vs. male rats, and the induction of expression in castrated males, but also show a significant increase of BP-1 expression in pregnant females, even in comparison with normal females or nursing females. Other sex-related hormones, such as progesterone, or glucocorticoids may also be involved in the expression of BP1-RNA.

Increased expression of the growth hormone receptor enhances growth by providing additional binding sites for either endogenous or exogenously administered growth hormone. This approach to growth enhancement is particularly useful in animals which are normally killed at an early age, such as chickens, before or just at the time the receptor is expressed at significant levels, or in males in which the receptor may not be fully expressed even in adulthood.

### EXAMPLES

### Example 1

Poly(A) mRNA is isolated from the livers of Sprague-Dawley rats by chromatography on oligo d(T) cellulose (Maniatis et al., Molecular Cloning. A Laboratory Manual. Cold Spring Harbor Laboratory, 1982). The poly(A)⁺ RNA is fractionated by electrophoresis in a 1% agarose gel containing formaldehyde and transferred to nylon filters (Nytran, Schleicher & Schnell) as described (Derman et al. Cell 23:731-739, 1981). Radioactive probes, labelled with [α³²-P]dCTP by random priming (Feinberg and Vogelstein, Anal. Biochem. 132:6-13, 1983; Anal. Biochem. 137:266-267, 1983), are prepared from the 5' untranslated regions of two cDNA clones of rat growth hormone binding protein, called pRat1 and pRat6 (ATCC 67848 and 67844), as described in EP 0 383 205, the contents of which are incorporated herein by reference. These cDNAs each contain a distinct 5' untranslated region, called BP1 and BP2, respectively (Baumbach et al., Genes Devel. 3:1199-1205, 1989, also incorporated herein by reference). Probes made from these two 5' untranslated regions are called 5'BP1 and 5'BP2, respectively. These probes are separately hybridized to the mRNA on filters, which are washed and exposed to X-ray film. Figure 1 shows the result of this (Northern) analysis for male and female adult rat liver mRNA. The 5'BP1 probe (derived from pRat1) hybridizes to two species of mRNA from female rat liver, but does not hybridize to these species from male rat liver. In contrast, the 5'BP2 probe (derived from pRat6) hybridizes equally to the same two species in both male and female rat liver. This result suggests that specific sequences control the transcription of a class of mRNA represented by 5'BP1 in a sex-specific manner. As these control sequences do not appear to affect transcription of mRNA hybridizing to 5'BP2, it can be deduced that the control sequences are cis-acting, and are probably found 5' (upstream) of the rat genomic sequence containing the 5' untranslated region found in pRat1 (BP1).

### Example 2

Poly A⁽⁺⁾ RNA is isolated from rat livers, fractionated, transferred to filters and hybridized to radioactive probes as in Example 1. Three identical preparations are compared, using the following different probes: 5'BP1 and 5'BP2 from Example 1; and Rat 1-20 which is derived from the coding region shared by the rat growth hormone binding protein (GHBP) and rat growth hormone receptor GHR) (EP 0 383 205). mRNA levels, as indicated by hybridization to the Rat 1-20 probe, represent the total amount of GHR and GHBP mRNA, including mRNA that contains various 5' or 3' untranslated regions. In contrast, levels of mRNA encoding GHR and GHBP that hybridize to either 5'BP1 or 5'BP2 probes each represent a subset of that total mRNA (Figure 2). As liver development proceeds from day 1 through day 28, total levels of GH receptor and binding protein mRNA gradually increase, and this pattern is similar with the 5'BP2 probe and the Rat 1-20 (coding region) probe. In contrast, the expression seen with the 5'BP1 probe is much less through day 28, yet in adult (female) rat, the levels of expression are very high. Thus, expression of the 5'BP1 region is even more strongly developmentally regulated than the total developmental regulation of GH receptor and binding protein message. It is first detected at about 4 weeks of age in both males and females, and quickly reaches in females the very high levels seen in the adult, whereas the low level seen in 4-week-old males does not appreciably increase with age. Levels of BP1 hybridizing message are uniformly high in pregnant females (Figure 2).

### Example 3

Using genomic DNA isolated from the liver of a Sprague-Dawley rat and partially digested with restriction endonuclease Sau 3A, a library is prepared in bacteriophage λGEM 11 (Promega). A clone, 5'λ1, is isolated by hybridization to the 5'BP1 probe and analyzed. An approximately 5-kilobase EcoRI fragment is subcloned in pGEM3zf⁽⁻⁾ and called pRat1E. This fragment contained the 5' untranslated region found in pRat1. Figure 3 shows the restriction map of pRat1E, with the sequenced region shown in Figure 4 highlighted. Inspection of the sequence reveals the following: all of the BP1 5' untranslated region is found (called (Exon 1); the point at which mRNA splicing joins this region to the rest of the GHR/GHBP sequence is deduced by loss of homology to BP1, and signals the beginning of Intron 1 (Figure 4).

The start site of transcription for Exon 1 is determined by primer extension analysis (Maniatis et al., 1989). Reverse transcription of female and male rat liver mRNA is primed by an oligonucleotide called BP1B, which is labelled at its 5' end with polynucleotide kinase, using γ³²P-ATP (New England Nuclear). This primer hybridizes to the RNA sequence underlined in Figure 4, and is extended by reverse transcription to the 3' end of the mRNA, indicating the transcriptional start site of using female liver mRNA and nothing is observed using male liver mRNA. The size of the bands is determined by running a sequencing reaction of pRat1E primed by the same oligonucleotide primer in the accompanying lanes. In Figure 4, stars underneath the sequence denote the bases determined to be transcriptional start sites. This location is thus shown in Figure 4 as the beginning of Exon 1.

### Example 4

Operated and sham-operated rats are 3 weeks of age at the time of operation, and are sacrificed at 7 weeks of age. Livers are flash frozen in liquid nitrogen and stored at -80^{o}C. RNA is prepared and poly(A) selected as in Example 1. 3 µg of poly(A) mRNa per lane is fractionated on 1% agarose-formaldehyde gels and transferred to Nytran filters (S&S). Filters are probed with a ³²P-labelled (T₇ Quick Prime, Pharmacia), 220 bp fragment of Rat BP1 (Shown in Figure 4) from the Pvu II site at position 30 to the Bg1 II site at position 259. Filters are hybridized in Church buffer and washed 3-5 times in 0.2 x SSC and 0.2% SDS at 65^{o}C. Since this fragment is derived from a genomic clone of BP1, cross contamination of coding region sequences is eliminated. Filters are decayed and additionally washed in boiling water containing 1% SDS, and reprobed with a 1 kb Pst I fragment of the chicken GAPDH gene. This gene encodes glyceraldehyde 3-P dehydrogenase, a "housekeeping" enzyme that is transcribed at constant levels, and is used to normalize RNA levels among samples on the same gel, thus allowing more accurate quantitation. Analysis is performed on a Molecular Dynamics Phorphorimager, and the quantitation of individual bands is performed using ImageQuant software (Molecular Dynamics). Data is presented as the ratio of GHBP RNA to GAPDH RNA. In female rats, high levels of this message are seen in both ovariectomized and sham-operated females, although a modest decrease occurs in the ovariectomized animals. Sham-operated males display nearly undetectable levels of this RNA, whereas a significant amount of RNA is observed in castrated males.

### Example 5

Rats are castrated or sham-castrated at 6 weeks of age and treated for 3 days two weeks later. Treatments consist of 50 µg or 10 µg per day of estrogen or testosterone (Sigma) in sesame oil (200 µl) injected intraperitoneally. Livers are processed, and RNA is prepared and analyzed as described in the previous Example. Analysis is performed on a Molecular Dynamics Phorphorimager, and quantitation of individual bands is performed using Image-Quant software (Molecular Dynamics). Data is presented as the ratio of GHBP RNA to GAPDH RNA. As shown in Figure 7, castrated animals have significantly more GHBP RNA than sham-castrated animals. Estrogen treatment of sham-castrated animals results in a modest increase of this RNA, whereas testosterone treatment probably has little effect. Testosterone treatment of castrated animals also has little effect, although there may be a trend toward reduced RNA levels with low levels (10 µg/day) of testosterone. Treatment of castrated animals with estrogen, however, dramatically increases the levels of GHBP RNA that are recognized with the BP-1 probe. These observations suggest that estrogen positively regulates the BP-1 promoter element, whereas testosterone may have a negative effect or none at all (high levels of testosterone may cross react with estrogen receptors).

### Example 6

Hypophysectomized (hypox) rats, castrated rats and controls are obtained from Taconic Farms, Germantown, NY. Treatments and sacrifice are performed when the rats are 7.5 weeks of age. Treatments are: estrogen, 50 µg in 0.2 ml sesame oil intraperitoneally; DHT, 50 µg in 0.2 ml sesame oil, intraperitoneally. Animals are sacrificed 8 hours after treatment (including vehicle-treated controls) unless specified in Figures.

In the first experiment, a time course of induction for BP-1 GHR and GHBP mRNA is determined with estrogen treatment, compared with 8-hour treatments with estrogen or DHT (a testosterone analog that is not converted in vivo to estrogen). Sham castrated rats are used as a control. Northern analysis is shown in Figure 8 (top), where BP-1 RNA is induced in castrated vs. sham castrated animals; estrogen treatment of sham castrated animals does not show marked induction; expression of BP-1 is marked in castrated animals with estrogen treatment over a period of 8 hours, with indications of induction as early as 1-2 hours. Comparison with Figure 8 (bottom), where a GHR/GHBP coding region probe detects all classes of GHR/GHBP mRNA, little difference is observed among all samples.

In the second experiment, hypox animals (both male and female) are compared with sham-hypox animals, and estrogen treatment (8 hours) is performed in hypox + castrated and sham-hypox + castrated animals. Northern analysis using the BP-1 probe is shown in Figure 9 (top): in normal male, castrated male and female rats, hypox animals display a marked lack of BP-1 expression, even with estrogen treatment; and estrogen induces BP-1 expression as expected in sham-hypox + castrated male rats. Again, as seen in Figure 9 (bottom), total RNA expression of GHR/GHBP using a coding region probe is not markedly affected by hypophysectomy. Thus, expression of the BP-1 class of RNA is specifically reduced in hypox rats, implying that an anterior pituitary-derived hormone may regulate this promoter. Candidates for this regulation are GH itself or other pituitary hormone such as prolactin, ACTH, LH, FSH or TSH.

### Example 7

A fragment of pRat1, a rat GHBP clone that includes the BP-1.5' untranslated region, is isolated by PCR. A probe is produced from this cloned fragment by cutting with BGL II (at position -24), and synthesizing RNA in the presence of radiolabelled CTP with T7 polymerase. This probe is hybridized with RNA from rat tissues, the annealed complex is treated with RNAase, purified and run on a denaturing polyacrylamide gel. The fragment spans the region -35 to +144 of BP-1 (GHR or GHBP) and the entire probe contains 67 bases at the T7 promoter end that are derived from the vector. Thus, the entire probe would be expected to contain 235 bases (24 + 144 + 67). The probe will be expected to protect the region -11 to +144 (155 bases) for all non-BP-1 GHR/GHBP RNA, and would be expected to protect the entire length (168 bases) of BP-1 RNA. Thus, as shown in Figure 10, the full length probe is reduced in size upon hybridization and digestion with RNAase to yield two protected bands of the correct sizes, 155 and 168 bases.

In Figure 10, RNA from the liver of male, castrated male, female and pregnant female rats as well as RNA from other rat tissues are compared using this assay. Essentially, these results confirm previous Northern analysis, demonstrating that BP-1 RNA is preferentially expressed in female vs. male rats, is mostly liver specific, and is induced upon castration of males. The RNA from pregnant rat liver shows a dramatic induction of this message, both compared with normal female liver and liver from nursing mothers. Thus, it appears that a pregnancy-related factor is involved in the induction of BP-1 RNA. These results, when combined with the hypox results (previous Example), imply either that reproduction-related pituitary hormones, such as FSH and LH (LH in particular may increase in late pregnancy), or other sex-related hormones besides estrogen, such as progesterone, may regulate the expression of BP-1 RNA in female rats.

### DEPOSIT OF BIOLOGICAL MATERIALS

The following biological materials have been deposited on December 13, 1991 with the American Type Culture Collection, 12301 Parklawn Drive, Rockville, MD.

| Strain | Accession No. |
|---|---|
| E. coli-K-12Dhα5/pRat1-E | ATCC 68879 |

Sequence ID No.: 1

Sequence Type: Nucleic Acid

Sequence Length: 1321 Base Pairs

Topology: Linear

Strandedness: Single

Original Source Organism: Rat

CTCAATTATT CTTTGATGGT CAGGTGAGCT GAGATGAAGG AATAAACCAA 50

TATTTACAGA ACACACTAAA GGTATTCCAA ATTAAAGATA CTATTACAAT 100

GAATGGTTGA AAGTCTACAA TAGAATGAAA TATACTAGGA ACTTCAATGC 150

ATAGTGGTAT TTCAGAATTT GTTTTTACAT TTATTTCTTC ATTTAAAAAA 200

ATGTTCAATG TTCTGGCACT CTGAGGCCTT GGGAAGCTTA AATTTTATAT 250

GAGTGGATTT CTGTTCTTGT TCTTACTCTT CTGAGAGGTG TCACAGTACC 300

ACATTAAAAT GACCCAGTTA TCCCTAGGTG CTGGTGGCAT ACCCCCTTAA 350

TCCAGCACTC AAGGCAGAGG CAAGTTTATC TTTGAGTTTG AGGCCAGCCT 400

GGTCTACAGA GTTCCAGAAC AGCCAAGGCC ACATAGAGAA ACCCTGTCTT 450

AAAAACACAA ACAACAACAA AAATATCCAA TTACTACATT CCATTTATTT 500

AGATGGAAAA CATTATCTGC CTCAAGCTCT AACATTCAGC TCTTCTTAAA 550

TGATCTCATG GTCTCAGGTA GACTTTAGCC CAGGTTGGCC TTCACTTCAG 600

CTGGAAGCAT CTAAAAGTGT TCTGGGCAAA GAGCACCAGC TGCATTAGAT 650

GAAGCAATCA TCTGGGACAA TTGATACACT CTTCTGGAAC CAGGTGTGTG 700

AGAGATAGTG GGAGAGACCA CAAGATAGAG GATTATTAAT TTATGGGGAT 750

TTTTTGGAGA TTTATATACC CAAGCAACAA CTCCTAGATG CTACAGACCA 800

AGACACCAAG AAATAACCAC GAAATTCTAC TTTCAACCCT AGCTTCTCTA 850

CCAGATTTAA CTAAACAAGA TCTTCTTGCA AAGGTAAATA AATGGAGAAA 900

AAAAGATTAT TGATATAAAT TCTGAAAGAT ATTGTAAGAC TATCCAGCCT 950

TCATTTCAGA GGTATCTACA GGACTGTTGG TTCGTATGGC TAACTTTGTT 1000

TAAATGTTCC TAAGTCAGAT CGGGTCTCTG TCACTACTCC TATTTGCTTT 1050

TGACTTTTAT AAAAAAAAAA AATTCTAACC AAGCTAAGCT TAGATTTATT 1100

CTTTGTTGTT GTTCCATTTT TTTATTTTTT TTCCTTTACC TAAATCTCTC 1150

TGTATCCGTC TTCTCTCTAA GTCTATCTTT TCCAAGTGCT GGAGAAGCCT 1200

TTGACCGCTT GCTCCTTTTA TCACTGAACC T 1231

## Claims

1. An isolated nucleic acid sequence comprising a region 5' to an untranslated region upstream of a gene encoding a growth hormone receptor.

2. The sequence of Claim 1 which is characterized by being
(a) capable of causing delayed expression of a gene under its control in vivo;
(b) capable of causing expression of a gene under its control only in females and castrated males.

3. The sequence of Claim 1 which also comprises all or a portion of the untranslated region.

4. The sequence of Claim 1 which comprises that portion of the nucleotide sequence of Figure 4 which is 5' to Exon 1, or a corresponding sequence isolated from a different species.

5. The sequence of Claim 4 which also comprises all or a portion of Exon 1.

6. The sequence of Claim 5 which also comprises all or a portion of Intron 1.

7. The sequence of Claim 1 which is contained in the strain deposited as ATCC 68879.

8. An isolated DNA construct comprising the sequence of Claim 1 operably linked to a gene other than a growth hormone receptor gene, or to a growth hormone gene.

9. A transgenic non-human animal comprising the construct of Claim 8.

10. A method for modulating expression of a gene product under control of the sequence of Claim 1 which comprises administering to an animal containing the sequence an effective amount of estrogen or estrogen-like compound.

11. A method for identifying compounds which are capable of modulating activity of a growth hormone receptor promoter which comprises contacting a microorganism containing the promoter linked to a reporter gene with a sample to be tested, and observing the presence or absence of expression of the reporter gene.
